# EUROPEAN PATENT SPECIFICATION

(11) **EP 2 600 914 B1**
(45) Date of publication and mention of the grant of the patent: **05.08.2015**
(21) Application number: 11743425.8
(22) Date of filing: 05.08.2011
(51) Int. Cl.: A61L 29/14, A61M 25/00

(54) **FLEXIBLE WATER VAPOUR BARRIER MULTILAYER TUBE FOR PACKAGING PURPOSE**
WASSERDAMPFSPERRSCHLAUCH FÜR VERPACKUNGSZWECKE
TUBE MULTICOUCHE FLEXIBLE ÉTANCHE À LA VAPEUR D'EAU UTILISABLE EN TANT QU'EMBALLAGE

(30) Priority: 05.08.2010 DK 201070350
(43) Date of publication of application: 12.06.2013
(73) Proprietor: Coloplast A/S, 3050 Humlebæk (DK)
(72) Inventor: NIELSEN, Henrik, Lindenskov, DK-2765 Smoerum (DK)
(86) International application number: PCT/DK2011/050304
(87) International publication number: WO 2012/016570

(56) References cited:
- EP-A1- 0 679 506
- WO-A1-97/47349
- WO-A1-98/19729
- WO-A1-99/30761
- GB-A- 2 319 507

## Description

### Field of the invention

The present invention relates to a multilayer tubular package for a urinary catheter. Furthermore, the invention relates to a catheter package set comprising a multilayer tubular package, a catheter and a swelling medium.

### Background

Urinary catheters are used as a tool assisting in the draining of the urinary bladder of persons that have reduced or non-existing bladder control. The reduced or non-existing bladder control may either be temporary or permanent, where a temporary loss of bladder control may be caused by trauma, loss of consciousness or illness, as an example. An example of a permanent loss of bladder control may be, where a loss of a neural connection between the brain or spinal cord and the urinary bladder occurs due to a trauma to the spinal cord, as is often the case with para- and tetraplegics.

One example of a urinary catheter which is widely used for draining urine from the urinary bladder, is where a catheter tube is inserted into the urethra of a user and where the tip of the catheter tube is maneuvered into the urinary bladder, forcing the urethral sphincter open and thus providing a drainage channel from the urinary bladder and out of the body, via the catheter tube. There are two types of catheters which are commonly used, the permanent catheter and the intermittent catheter. The permanent catheter is a highly flexible catheter which is inserted by medical professionals into the body for a long period of time and where the catheter is anchored inside the bladder. The intermittent catheter is usually a single use catheter or a multiple use catheter, which is inserted by the user into the urethra/bladder for the immediate drainage of their urinary bladder and is removed from the urethra/bladder subsequent to the drainage.

There are a number of different types of intermittent catheters which are currently available for the user, such as the SpeediCath® and EasiCath® marketed by Coloplast A/S which are conventional one-piece catheter tubes which have an outlet at its distal end which may be used to connect the catheter to a urinary bag for collecting the urine drained from the urine bladder.

Catheters are usually coated with a hydrophilic coating. The coating is as a minimum applied on that part of the surface which is introduced or comes into contact with e.g. mucous membranes during introduction of the device. Whereas such a coating is not particularly slippery when dry, it may become extremely slippery when it is swelled with water before introduction into the human body. The hydrophilic coating thus ensures a substantially painless introduction with a minimum of damage on tissue.

Ready to use hydrophilic coated intermittent catheters are usually packed with a swelling medium such as water and optionally containing various additives.

Some catheter packaging concepts are based on tubes instead of packaging films or foils.

The problem has until now been to secure that such tube is flexible and at the same time gives satisfactory shelf life for the product.

Conveen® Expect catheter, the first ever ready to use hydrophilic coated intermittent catheter, was launched in year 2000 by Coloplast A/S. This product was packed in corrugated tubing made of 14% polyethylene vinyl acetate copolymer (EVA) material. The tube had good flexibility but had poor water barrier properties. Therefore, it was delivered with an aluminium bag as a secondary packaging.

SpeediCath® Compact Female catheter was launched in 2004. This product was primarily packed in a 1 mm thick polypropylene (PP) homopolymer tubing which gives the required water barrier. However, this tube has no flexibility.

EP 2,106,821 discloses a catheter assembly comprising a catheter having an insertion end and a discharge end, and a receptacle. The receptacle comprises a tubular member which in a storage state accommodates a catheter section including the insertion end of the catheter. The tubular member has at least one pleated region located along the tubular member arranged with a plurality of pleats, which pleats in the storage state forms a curvature, thereby enabling for a curved disposition of a catheter section located therein. The catheter assembly allows the catheter to, during storage, be curved such that minimum space consumption is required, meanwhile kinking of the catheter is avoided.

WO 2007/050685 discloses a pre-wetted intermittent catheter apparatus including a collapsible container. The catheter apparatus includes a container made of a gas impermeable material such as polypropylene and polyethylene. The pleat or folds in the outer surface of the container permit the container to compress or collapse.

EP0679506 A discloses a packaging material for a catheter comprising a first film of flexible polyethylene material with a water vapour transmission rate of less than 20 grams/m2/24 hours and a second film of polyester material.

From the above examples it can be seen that there is a need at the same time to fulfill the water barrier and the flexibility properties, when packaging ready to use hydrophilic coated catheters in a tube package.

### Summary of the invention

The present invention provides a flexible multilayer tubular package. The package is for an intermittent catheter. The intermittent catheter is coated with a hydrophilic coating, which becomes slippery when in contact with a swelling medium. Thus, the tubular package of the invention can withhold the swelling medium for a long time and at the same time being flexible and bendable.

Furthermore, the present invention is providing a catheter package set, which comprises a tubular package, an intermittent catheter and a swelling medium.

### Brief description of the drawings

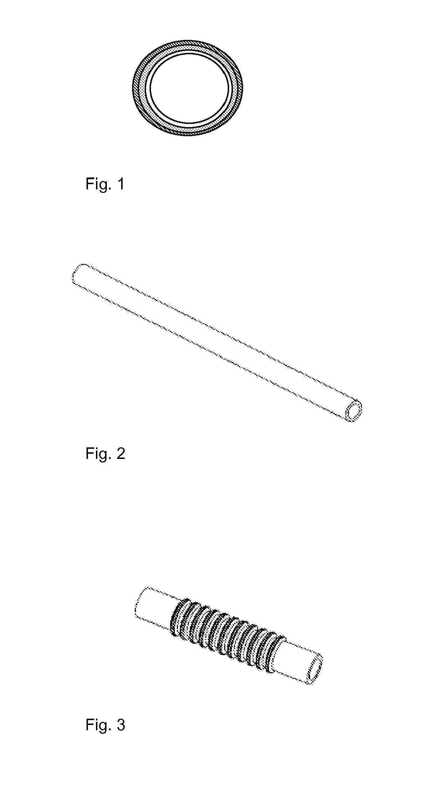

Figure 1 illustrates a multilayer tube according to the invention made of two or more individual material layers.
Figure 2 illustrates one embodiment of the invention, wherein the tube is straight.
Figure 3 illustrates an embodiment of the invention, wherein the tube is corrugated.

### Detailed description of the present invention

Abbreviations and Classification of materials:

| Trade name/ trivial name/ abbreviation | Chemical name |
|---|---|
| **Thermoplastic polymers:** | |
| ***Polyolefin based:*** | |
| PE | Polyethylene (either homopolymer or copolymer) |
| LDPE | Low density polyethylene (homopolymer) |
| LLDPE | Linear low density polyethylene (including copolymers with butene, hexene and octene) |
| HDPE | High Density Polyethylene |
| PP | Polypropylene, homopolymer |
| PP-cop. | Polypropylene copolymers including e.g. Vistamaxx from ExxonMobile |
| EVA | Poly-ethylene-vinyl-acetate copolymer |
| EMA | Poly-ethylene-methyl-acrylate copolymer |
| EEA | Poly-ethylene-ethyl-acrylate copolymer |
| EBA | Poly-ethylene-butyl-acrylate copolymer |
| COC | Cyclic polyolefin, e.g. Topas from Ticona |
| COC-PE | Compound of cyclic polyolefin and polyethylene |

| ***Halogen based:*** | |
|---|---|
| PVC | Polyvinyl Chloride |
| PVDC | Polyvinylidene Chloride, e.g. Selar from Dow Chemical |
| PCTFE | Polychlortriflourethylene e.g. Aclar from Honeywell |

| **Thermoplastic Elastomers:** | |
|---|---|
| ***Styrene based*:** | |
| SiBS | Polystyrene-*block*-poly(isobutylene)-*block*-polystyrene, e.g. Sibstar from Kaneka |
| SEBS | Polystyrene-*block*-poly(ethylene/butylene)-*block*-polystyrene, e.g. Kraton G from Kraton |
| SEPS | Polystyrene-*block*-poly(ethylene/propylene)-*block*-polystyrene, e.g. Septon from Kuraray |
| SBS | Polystyrene-*block*-poly(butadiene)-*block*-polystyrene, e.g. Kraton D (SBS types) |
| SIBS | Polystyrene-*block*-poly(Isoprene/butadiene)-*block*-polystyrene, e.g. Kraton D (SIBS types) |
| SIS | Polystyrene-*block*-poly(Isoprene)-*block*-polystyrene, e.g. Kraton D (SIS types) |

| ***Other polyolefin:*** | |
|---|---|
| PIB | Polyisobutylene |
| IIR | Uncured butyl rubber (poly-isobutylene-isoprene copolymer, e.g. Lanxess Butyl) |
| PP/EPDM | Polypropylene with in-situ crosslinked ethylene-propylene-diene-methylene rubber e.g. Santoprene from ExxonMobile Chemical |

| ***Halogen based:*** | |
|---|---|
| CIIR | Chlorobutyl uncured rubber |
| BIIR | Bromobutyl uncured rubber |

The aim of the invention is to secure that a tubular package for an intermittent catheter is flexible and bendable and at the same time gives satisfactory shelf life for the product. The goal is achieved by the choice of the tube material.

By tube material and the properties thereof is meant the total layered construction of the multilayer tube and the properties thereof.

A good shelf life of the products is achieved by keeping the catheter wet by the swelling medium at all times. Thus, the package should avoid leakage of any kind of the swelling medium from the package.

In an embodiment of the invention, a catheter package is formed as a tube for an intermittent catheter, wherein the tube material is flexible and has a low water vapour transmission rate. By tube or tubular is meant a cylinder-like element with a substantially circular cross section.

The tube material has a water vapour transmission rate below 0.50 g/m²/mm/24h, preferably below 0.30 g/m²/mm/24h.

Water vapour transmission rate of the tube material can be measured according to standard method ASTM E96.

By a multilayer construction is meant a construction with two or more individual material layers. This gives an extended possibility to optimize the tube properties with regard to many important parameters like:
- Water vapour barrier
- Water vapour transmission rate
- Flexibility
- Bending stiffness
- Kink resistance
- Price
- Welding properties (special welding layers on inside or outside)
- Surface friction
- Visual appearance
- Printing on outside
- Colouring

The catheter package of the invention is based on tubes instead of packaging films or foils. This gives advantages in making products which are very compact and discrete. The tubular product is easy to have in the pocket or bag. Moreover, the product is easier to handle and is more visually appealing.

Furthermore, a tubular package may give other product features and benefits such as using the tubular package as an extender tube to the toilet. For this intended use, it is also important that the tube has a certain flexibility and kink resistance.

It is also a requirement that the package can be radiation sterilized without degradation of the material and with only very small change of the geometrical dimensions.

As the standard solution today is to use a packaging based on aluminum foil, the package of the invention could also give an environmental advantage.

The packages disclosed in the prior art do not fulfill both the requirements with regards to barrier properties and to flexibility properties. Either the packages were using hard water barrier polymers like PP and HDPE in a thickness of 0.5 to 1.0 mm or they were soft as e.g. EVA and needed an extra secondary packaging to obtain the water vapour barrier requirements.

The barrier properties alone can be fulfilled by standard materials as PE or PP or more special materials COC, PVDC or Fluor polymers, however these are not very flexible and some of them have high cost and a poor environmental profile.

The flexibility properties can be fulfilled by using PE copolymers like EVA, EMA, EEA, EBA, or metallocene PP copolymers (like Vistamaxx; Exxon Chemical and, Versify; Dow Chemical) or by thermoplastic elastomer materials like SBS, SEBS, SEPS compounds or EPDM/PP compounds. Some of these have water vapour barrier properties to some extent. However for packaging of hydrophilic coated catheter, they have not at the same time been fulfilling the requirements for shelf life, flexibility, kink resistance, sterilization stability, environment and cost price.

Standard polyolefins like PE or PP have rather good water vapour barrier properties. However to get the water vapour barrier properties, it must be very unpolar (having essentially no polar co-monomers), and be very high crystalline. This together with the required wall thickness makes the package very inflexible.

For these unpolar polyolefins the barrier properties are well correlated with the percentage of crystalinity. For PP the crystalinity is mainly dependent of the ethylene co-monomer content and for some block copolymer types also on the polymerization process. For PE polymers the crystalinity is mainly depending on the branching defined polymerization process and on the content of unpolar co-monomers like butylenes, hexene and octene. PE is normally divided into 3 main categories, HDPE, LDPE and LLDPE. HDPE is quite linear and LDPE are more branched in structure. LLDPE is very linear in overall structure, but has the small scale branching defined by the co-monomers. For the same density LDPE and LLDPE are very similar in water vapour barrier and in flexibility. However, LLDPE has a higher melting point and thus better high temperature performance, which may be important in the sterilization process.

According to an embodiment of the invention, the composition of the tube material comprises polyethylene which is a low density polyethylene or a linear low density polyethylene.

By low density polyethylene (LDPE) is meant a thermoplastic polymer (polyethylene), wherein the LDPE is defined by a density range of 0.910 - 0.940 g/cm³.

By linear low density polyethylene (LLDPE) is meant a substantially linear polymer (polyethylene), with significant numbers of short branches, commonly made by copolymerization of ethylene with longer-chain olefins. Linear low-density polyethylene differs structurally from conventional low-density polyethylene because of the absence of long chain branching.

Standard PE's and PP's are too stiff for a straight flexible tubing. However some specialty grades of metallocene LLDPE/VLDPE and PP-PE copolymers with modulus below 100 MPa can be used. Examples of these materials are Clearflex (LLDPE from Polymeri) with densities below 0.91 g/cm³ and Vistamaxx and Versify (metallocene PP/PE copolymers from Exxon and Dow Chemical). For corrugated tubing good flexibility can be obtained for standard PE's with E-modulus below 200MPa. However, the barrier will be reduced because of a higher surface area of a corrugated tube (typically double area) and thinner material (typically half thickness).

In an embodiment of the invention, the layer composition of the tube comprises the combination of one or more thermoplastic polymer layer(s) and one or more thermoplastic elastomer layer(s).

In another embodiment of the invention, the layer composition of the tube comprises two or more thermoplastic polymer layers.

In another embodiment of the invention, the layer composition of the tube comprises two or more thermoplastic elastomer layers.

According to one embodiment of the invention, the tube is a 3-layer construction which comprises a thermoplastic polymer layer as an outer layer and as an inner layer and a thermoplastic elastomer layer as the middle layer.

According to another embodiment of the invention, the tube is a 3-layer construction which comprises a thermoplastic elastomer layer as an outer layer and as an inner layer and a thermoplastic polymer layer as the middle layer.

According to another embodiment of the invention, the tube is a 3-layer construction which comprises a thermoplastic elastomer layer as an outer layer and as an inner layer and another thermoplastic elastomer layer as the middle layer.

According to another embodiment of the invention, the tube is a 3-layer construction which comprises a thermoplastic polymer layer as an outer layer and as an inner layer and another thermoplastic polymer layer as the middle layer.

According to another embodiment of the invention, the tube is a 2-layer construction which comprises a thermoplastic elastomer layer as an outer layer and a thermoplastic polymer layer as the inner layer.

According to another embodiment of the invention, the tube is a 2-layer construction which comprises a thermoplastic elastomer layer as an outer layer and another thermoplastic elastomer layer as the inner layer.

According to another embodiment of the invention, the tube is a 2-layer construction which comprises a thermoplastic polymer layer as an outer layer and a thermoplastic elastomer as the inner layer.

According to another embodiment of the invention, the tube is a 2-layer construction which comprises a thermoplastic polymer layer as an outer layer and another thermoplastic polymer layer as the inner layer.

In an embodiment of the invention, the thermoplastic polymer layer(s) comprises polyethylene homopolymers with a density of 0.900-0.930 g/cm³.

According to one embodiment of the invention, the thermoplastic polymer layer(s) comprises polyethylene copolymers with butene, hexene or octene as co-monomers and with a density of 0.900-0.930 g/cm³.

In an embodiment of the invention, the thermoplastic polymer layer(s) comprises a polypropylene copolymer with from 1 % to 25% of ethylene as co-monomer either randomly distributed or as discrete blocks and with a density of 0.860-0.910 g/cm³.

According to another embodiment of the invention, the thermoplastic polymer layer(s) comprises polyethylene copolymers or terpolymers with from 1% to 25% of vinylactetate methylacrylate, ethylacrylate, butylacrylate, acrylic acid, methacrylic acid or maleic acid as comonomers.

The thermoplastic elastomer layer(s) may comprise elastomer as pure elastomers or as compounds.

Likewise, the thermoplastic polymer layer(s) may comprise polymer as pure polymers or as compounds.

A LLDPE with a density between 0,900 g/cm³ and 0,930 g/cm³ is a suitable compromise between flexibility, water vapour barrier and dimensional stability at radiation sterilization temperatures for the thermoplastic polymer layer.

In one embodiment of the invention, the thermoplastic elastomer layer(s) comprises styrenic elastomers as pure polymers or as compounds.

Thermoplastic elastomer compounds can be very soft and unpolar compounds. Such compounds may be based on SEBS together with PP or PE and in many cases with paraffin oil as a plasticizer. A customized compound can give the required flexibility, kink-resistance and water vapour barrier for a straight tube, and in the best cases it can also fulfill the water vapour barrier requirements for a corrugated tube.

According to an embodiment of the invention, the thermoplastic elastomer layer(s) further comprises polypropylene and/or polyethylene, and optionally a plasticizer such as paraffin oil in a compound.

In one embodiment of the invention the composition of the tube material contains 1-10% (w/w) of low molecular PE wax (Mw 500 to 1500 g/mol) in order to improve the barrier properties and flexibility.

By polyethylene wax (PE wax) is meant a low molecular weight polyethylene polymer that because of its low molecular weight has wax like physical characteristics.

Polyisobutylene is a polyolefin, which at the same time is soft and has very good water barrier properties, even much better than could be expected from its unpolar nature and low crystallinity. It could be explained by a molecular structure, which gives very low mobility of the polymer chains in the amorphous phase, even though the actual temperature is above the Tg for the amorphous phase of the material.

In its pure form polyisobutylene is not a solid material, thus it needs to be used either as:
- Cured or uncured butyl rubber (poly-isobutylene-isoprene copolymer e.g. Exxon Butyl, Lanxess Butyl).
- Block copolymer with e.g. polystyrene as the end blocks, SiBS, styrene-isobutylene-styrene (e.g. Sibstar from Kaneka).
- Compound with a thermoplastic material like polyethylene or polypropylene or styrene block-copolymers (e.g. SEBS, SEPS or SBS). These compound may contain polybutene, polyisobutylene, cured or uncured butyl rubber, and/or SiBS blockcopolymer.

According to one embodiment of the invention, the thermoplastic elastomer layer(s) comprises polybutene, polyisobutylene, cured or uncured butyl rubber, or block copolymer with polystyrene as the end blocks such as styrene-isobutylene-styrene.

According to another embodiment of the invention, the thermoplastic elastomer layer(s) comprises a styrene block copolymer such as polystyrene-*block*-poly(ethylene/butylene)-*block*-polystyrene, polystyrene-*block*-poly(ethylene/propylene)-*block*- polystyrene, polystyrene-*block*-polybutadiene-*block*-polystyrene or polystyrene-*block*-polyisobutylene-*block*-polystyrene.

According to an embodiment of the invention, the tube is straight.

By a straight tube is meant a tube without pleats.

According to another embodiment of the invention, the tube is corrugated.

By a corrugated tube is meant a tube with a series of pleats or parallel ridges and furrows.

According to an embodiment of the invention, the construction can also include the use of special tie-layers to secure adequate adhesion between the tube layers.

The tube package will normally be made in slightly more than the full length of the catheter, but can for certain applications be made in lengths up to e.g. 2 times, or even more, of the length of the catheter.

The tube can be closed in both ends with a hot melt plug. The tubes can as well be closed by other secure means e.g. by welded or glued small caps or lids or simply closing the ends by welding the tube together.

In an embodiment of the invention, a catheter package set comprises a tube package, an intermittent catheter, a swelling medium and optionally a urine collection bag, wherein the tube material is flexible and has a low water vapour transmission rate.

In one embodiment of the invention, the swelling medium is disposed within the tube package.

According to an embodiment of the invention, the catheter package set comprises a tube package as described above.

According to one embodiment of the invention, the catheter package set comprises an intermittent catheter comprising a hydrophilic coating.

In one embodiment of the invention, the package set has been sterilized using irradiation while in contact with the swelling medium.

In a preferred embodiment of the invention, the set is sterilized using β- or γ-irradiation.

### Experimental

### Materials tested

| | |
|---|---|
| EVA grade tested were: | Greenflex ML40 (14% vinyl acetate content) |
| LDPE grades tested were: | Riblene FH10 from Polimeri Europe |
| SEBS compound were: | Meliflex M6608 and R2783 from Melitek |
| SIBS were | Kraton DF-1170BT |
| SiBS were: | Sibstar T102 from Kaneka |

### Methods

Water permeability: The Water Vapour Transmission Rate (WVTR) at relevant environmental conditions. Standard measuring conditions often used in the literature 38°C with 90%RH difference, however 30°C and 40°C are commonly also used. The conditions used for the examples here are 30°C with 70% Relative Humidity difference (100%RH inside and 30%RH outside). For flat samples the permeability is linear correlated with the surface area and with the reciprocal thickness of the sample. For tube samples a good and simple approximation has been made for the calculation as follows. For straight as well as corrugated tubing the values has been calculated by using the mean of the inside and the outside surface area and the mean material thickness. The calculations of the equivalent permeability for the actual monolayer and multilayer constructions have been made on the results of the Water loss measurements, which have been performed on actual tube samples. See below.

Water loss: The water loss by permeation through the packaging is a combination of the actual geometrical design, the materials and the environmental conditions. The permitted water loss is depending on the product design. For the water loss measurements the actual tubes in a length of 350 mm has been filled with 6 ml of the actual catheter swelling media (93% water 6% PVP polymer and 0,9% NaCl) and, for the testing, closed in both ends with a hot melt plug of a minimum of 4 mm in thickness. By weighing the samples a minimum of 3 times within a few months of storage at 30°C and 70%RH difference a very good linear correlation can be made between the water loss and the storage time.

Flexibility: Subjective ability to be bended. For practical purposes the bending characteristics are correlated with the E modulus of the material and the geometrical size and shape. For relevant packaging tube sizes with inside diameter of 6 to 10 mm and wall thickness between 0.5 and 1.0 mm E-modulus up to 150 MPa can be used for straight tubing and up to 400 MPa for a tube with a corrugated design.

Sterilization: The catheter product is to be E-beam or Gamma sterilized.

### Water loss measurement

350mm length of the tubes were closed in one end with polyethylene hotmelt and packed with 6 ml of the PVP catheter swelling medium before closing the other end with the PE hotmelt. The products were weighed on a calibrated scale with an accuracy of +/-0.1 mg. Results reported for each experiment are average for 3 measurements, and they have been corrected for the weight loss for reference samples without the swelling medium.

### Example 1:

### 3-layer straight tube

Inside diameter: 4.9 mm; Outside diameter 6.1 mm
Layer thicknesses: Inner layer: 0.1 mm; Middle layer: 0.4 mm: Outer layer: 0.1 mm
Outside Surface Area: 40.9 cm²

### Water loss

| ID No. | Inner Layer | Middle Layer | Outer Layer | Weight loss, 6 days | Weight loss, 15 days | Weight loss, 32 days | Calculated Weight Loss 2 years (Linear regression) | Equivalent Permeability Coefficient 30°C with 85%RH difference |
|---|---|---|---|---|---|---|---|---|
| | | | | g | g | g | g | g/m²/mm/24h |
| 1 | Greenflex ML40, EVA | | | 0,090 | 0,224 | 0,438 | 9,7 | 1,95 |
| 4 | Riblene FH10, LDPE | | | 0,010 | 0,024 | 0,043 | 0,9 | 0,18 |
| 13 | Meliflex M6608, SEBS-Compund | | | 0,020 (5 days) | 0,080 (18 days) | 0,122 (30 days) | 3,0 | 0,60 |
| 3 | Greenflex ML40 EVA | Kraton DF-1170 BT, SIBS | Greenflex ML40 EVA | 0,096 | 0,336 | 0,604 | 13,9 | 2,79 |
| 6 | Riblene FH10 LDPE | Kraton DF-1170 BT, SIBS | Riblene FH10 LDPE | 0,012 | 0,044 | 0,078 | 1,8 | 0,36 |
| 8 | Meliflex M6608 SEBS-Compound | Sibstar 102T, SiBS | Meliflex M6608 SEBS-Compound | 0,011 (5 days) | 0,044 (18 days) | 0,069 (30 days) | 1,7 | 0,34 |
| 9 | Greenflex ML40 EVA | Sibstar 102T, SiBS | Meliflex M6608 SEBS-Compound | 0,011 (5 days) | 0,047 (18 days) | 0,074 (30 days) | 1,8 | 0,37 |
| 10 | Greenflex ML40 EVA | Sibstar 102T, SiBS | Riblene FH10 LDPE | 0,010 (5 days) | 0,040 (18 days) | 0,061 (30 days) | 1,5 | 0,30 |

### Flexibility

| ID No. | Inner Layer | Middle Layer | Outer Layer | Modulus of Middle Layer ISO 527 | Shore A hardness of Middle Layer | Flexibility | Kink |
|---|---|---|---|---|---|---|---|
| | | | | MPa | | Actual Construction | |
| 1 | Greenflex ML40 EVA | | | 60 MPa | 92 Shore A | Acceptable | Acceptable |
| | | | | | 40 Shore D | | |
| 4 | Riblene FH10 LDPE | | | 160 MPa | 50 Shore D | Too Stiff | Acceptable for some applications |
| 13 | Meliflex M6608 SEBS-Compund | | | 60 MPa | 60 MPa | Acceptable | Acceptable |
| 3 | Greenflex ML40 EVA | Kraton DF-1170 BT, SIBS | Greenflex ML40 EVA | Shore A 44 | Shore A 44 | Acceptable and very flexible | Acceptable |
| | | | | 8 MPa (300% modulus) | 8 MPa (300% modulus) | | |
| 6 | Riblene FH10 LDPE | Kraton DF-1170 BT, SIBS | Riblene FH10 LDPE | Shore A 44 | Shore A 44 | Acceptable | Acceptable for some applications |
| | | | | 8 MPa (300% modulus) | 8 MPa (300% modulus) | | |
| 8 | Meliflex M6608 SEBS-Compound | Sibstar 102T, SiBS | Meliflex M6608 SEBS-Compound | 0,5 MPa (100% modulus) | 0,5 MPa (100% modulus) | Acceptable and very flexible | Acceptable |
| 9 | Greenflex ML40 EVA | Sibstar 102T, SiBS | Meliflex M6608 SEBS-Compound | 0,5 MPa (100% modulus) | 0,5 MPa (100% modulus) | Acceptable and very flexible | Acceptable |
| 10 | Greenflex ML40 EVA | Sibstar 102T, SiBS | Riblene FH10 LDPE | 0,5 MPa (100% modulus) | 0,5 MPa (100% modulus) | Acceptable and very flexible | Acceptable |

### Discussion

From the examples, it can be seen that a satisfactory compromise can be obtained between the water loss and flexibility for a multilayer tube.

From ID No. 1, 4 and 13 it can be seen that none of these thermoplastic polymers or thermoplastic elastomers fulfills all the requirements for water loss, flexibility and kink resistance when made as a monolayer tube. However, when these materials are used in a multilayer tube in combination together with thermoplastic elastomers good results can be obtained in some cases as seen for ID No. 6, 8, 9 and 10.

It is an object of the present invention that when having this possibility also other properties such as welding properties, surface friction, visual appearance and cost price for the tube can be optimized by making the proper choice of material combination and layer thicknesses.

## Claims

1. A catheter package formed as a multilayer tube for an intermittent catheter, wherein the tube is a cylinder-like element with a substantially circular cross section and the tube material is flexible and has an equivalent water vapour transmission rate below 0.50 g/m²/mm/24h as measured with the WVTR method as defined in the description.

2. The catheter package according to claim 1, wherein the tube material has an equivalent water vapour transmission rate below 0.30 g/m²/mm/24h as measured with the WVTR method as defined in the description.

3. The catheter package according to any of the preceding claims, wherein the layer composition of the tube comprises the combination of one or more thermoplastic polymer layer(s) and one or more thermoplastic elastomer layer(s).

4. The catheter package according to any of the preceding claims, wherein the layer composition of the tube comprises two or more thermoplastic polymer layers.

5. The catheter package according to any of the preceding claims, wherein the layer composition of the tube comprises two or more thermoplastic elastomer layers.

6. The catheter package according to claims 3 and 5, wherein the thermoplastic elastomer layer(s) comprises a styrene block copolymer such as polystyrene-*block-*poly(ethylene/butylene)-*block*-polystyrene, polystyrene-*block*-poly(ethylene/propylene)-*block*-polystyrene, polystyrene-*block*-polybutadiene-*block*-polystyrene or polystyrene-*block-*polyisobutylene-*block*-polystyrene.

7. The catheter package according to claim 6, wherein the thermoplastic elastomer layer(s) further comprises polypropylene and/or polyethylene, and optionally a plasticizer such as paraffin oil in a compound.

8. The catheter package according to any of claims 3-7, wherein the thermoplastic elastomer layer(s) comprises polybutene, polyisobutylene, cured or uncured butyl rubber, or block copolymer with polystyrene as the end blocks such as styrene-isobutylene-styrene.

9. The catheter package according to any of claims 3-7, wherein the thermoplastic polymer layer(s) comprises polyethylene homopolymers with a density of 0.900-0.930 g/cm³.

10. The catheter package according to any of claims 3-7, wherein the thermoplastic polymer layer(s) comprises polyethylene copolymers with butene, hexene or octene as comonomers and with a density of 0.900-0.930 g/cm³.

11. The catheter package according to any of claims 3-7, wherein the thermoplastic polymer layer(s) comprises a polypropylene copolymer from 1% to 25% of ethylene as comonomer either randomly distributed or as discrete blocks and with a density of 0.860-0.910 g/cm³.

12. The catheter package according to any of claims 3-7, wherein the thermoplastic polymer layer(s) comprises polyethylene copolymers or terpolymers from 1% to 25% of vinylactetate, methylacrylate, ethylacrylate, butylacrylate, acrylic acid, methacrylic acid or maleic acid as comonomers.

13. The catheter package according to any of the claims 9 or 10, wherein the polyethylene is a low density polyethylene or a linear low density polyethylene.

14. The catheter package according to any of the preceding claims, wherein the tube is corrugated.

15. The catheter package according to any of the claims 1-13, wherein the tube is straight.

16. A catheter package set comprising
- a tube package
- an intermittent catheter
- a swelling medium,
- and optionally a urine collection bag,
wherein tube is a cylinder-like element with a substantially circular cross section and the tube material is flexible and has a water vapour transmission rate below 0.50 g/m²/mm/24h as measured with the WVTR method as defined in the description.

17. The catheter package set according to claim 16, wherein the swelling medium is disposed within the tube package.

18. The catheter package set according to any of the claims 16-17, wherein the tube package is according to any of the claims 1 to 15.

19. The catheter package set according to any of the claims 16-18, wherein the intermittent catheter comprises a hydrophilic coating.

20. The catheter package set according to any of the claims 16-19, wherein the package set has been sterilized using irradiation while in contact with the swelling medium.

21. The catheter package set according to claim 20, wherein the set is sterilized using β-or y-irradiation.

## Patentansprüche

1. Katheterverpackung, gebildet als mehrschichtiger Schlauch für einen intermittierenden Katheter, wobei der Schlauch ein zylinderartiges Element mit einem im Wesentlichen kreisförmigen Querschnitt ist und das Schlauchmaterial flexibel ist und eine äquivalente Wasserdampftransmissionsrate von unter 0,50 g/m²/mm/24 h, wie gemessen durch das in der Beschreibung definierte WVTR-Verfahren, aufweist.

2. Katheterverpackung gemäß Anspruch 1, wobei das Schlauchmaterial eine äquivalente Wasserdampftransmissionsrate von unter 0,30 g/m²/mm/24 h, wie gemessen durch das wie in der Beschreibung definierte WVTR-Verfahren, aufweist.

3. Katheterverpackung gemäß einem der vorstehenden Ansprüche, wobei die Schichtzusammensetzung des Schlauchs die Kombination von einer oder mehreren thermoplastischen Polymerschicht(en) und einer oder mehreren thermoplastischen Elastomerschicht(en) umfasst.

4. Katheterverpackung gemäß einem der vorstehenden Ansprüche, wobei die Schichtzusammensetzung des Schlauchs zwei oder mehr thermoplastische Polymerschichten umfasst.

5. Katheterverpackung gemäß einem der vorstehenden Ansprüche, wobei die Schichtzusammensetzung des Schlauchs zwei oder mehr thermoplastische Elastomerschichten umfasst.

6. Katheterverpackung gemäß Ansprüchen 3 und 5, wobei die thermoplastischen Elastomerschicht(en) ein Styrol-Blockcopolymer umfassen, wie z. B. Polystyrol-*block*-poly(ethylen/butylen)-block-polystyrol, Polystyrol-*block-*poly(ethylen/propylen)-*block*-polystyrol, Polystyrol-*block*-polybutadien-*block*-polystyrol oder Polystyrol-*block*-polyisobutylen-*block-*polystyrol.

7. Katheterverpackung gemäß Anspruch 6, wobei die thermoplastischen Elastomerschicht(en) ferner Polypropylen und/oder Polyethylen und gegebenenfalls einen Weichmacher, wie z. B. Paraffinöl in einer Verbindung, umfassen.

8. Katheterverpackung gemäß einem der Ansprüche 3-7, wobei die thermoplastischen Elastomerschicht(en) Polybuten, Polyisobutylen, gehärteten oder nichtgehärteten Butylgummi oder Blockcopolymer mit Polystyrol als Endblöcke, wie z. B. Styrol-Isobutylen-Styrol, umfassen.

9. Katheterverpackung gemäß einem der Ansprüche 3-7, wobei die thermoplastischen Polymerschicht(en) Polyethylen-Homopolymere mit einer Dichte von 0, 900-0, 930 g/cm³ umfassen.

10. Katheterverpackung gemäß einem der Ansprüche 3-7, wobei die thermoplastischen Polymerschicht(en) Polyethylen-Copolymere mit Buten, Hexen oder Octen als Comonomere und mit einer Dichte von 0,900-0,930 g/cm³ umfassen.

11. Katheterverpackung gemäß einem der Ansprüche 3-7, wobei die thermoplastischen Polymerschicht(en) ein Polypropylen-Copolymer mit 1 % bis 25 % an Ethylen als Comonomer, entweder wahllos verteilt oder als diskrete Blöcke, und mit einer Dichte von 0,860-0,910 g/cm³ umfassen.

12. Katheterverpackung gemäß einem der Ansprüche 3-7, wobei die thermoplastischen Polymerschicht(en) Polyethylen-Copolymere oder Terpolymere mit 1 % bis 25 % an Vinylacetat, Methylacrylat, Ethylacrylat, Butylacrylat, Acrylsäure, Methacrylsäure oder Maleinsäure als Comonomere umfassen.

13. Katheterverpackung gemäß einem der Ansprüche 9 oder 10, wobei das Polyethylen ein Polyethylen mit niedriger Dichte oder ein lineares Polyethylen mit niedriger Dichte ist.

14. Katheterverpackung gemäß einem der vorstehenden Ansprüche, wobei der Schlauch gewellt ist.

15. Katheterverpackung gemäß einem der Ansprüche 1-13, wobei der Schlauch gerade ist.

16. Katheterverpackungszusammenstellung umfassend
- eine Schlauchverpackung,
- einen intermittierenden Katheter,
- ein quellendes Medium,
- und gegebenenfalls einen Urinsammelbeutel,
wobei der Schlauch ein zylinderartiges Element mit einem im Wesentlichen kreisförmigen Querschnitt ist und das Schlauchmaterial flexibel ist und eine Wasserdampftransmissionsrate von unter 0,50 g/m²/mm/24 h, wie gemessen durch das wie in der Beschreibung definierte WVTR-Verfahren, aufweist.

17. Katheterverpackungszusammenstellung gemäß Anspruch 16, wobei das quellende Medium innerhalb der Schlauchverpackung angeordnet ist.

18. Katheterverpackungszusammenstellung gemäß einem der Ansprüche 16-17, wobei die Schlauchverpackung gemäß einem der Ansprüche 1 bis 15 ist.

19. Katheterverpackungszusammenstellung gemäß einem der Ansprüche 16-18, wobei der intermittierende Katheter einen hydrophilen Überzug umfasst.

20. Katheterverpackungszusammenstellung gemäß einem der Ansprüche 16-19, wobei die Verpackungszusammenstellung in Kontakt mit dem quellenden Medium unter Verwendung von Bestrahlung sterilisiert worden ist.

21. Katheterverpackungszusammenstellung gemäß Anspruch 20, wobei die Zusammenstellung unter Verwendung von β- oder γ-Strahlung sterilisiert ist.

## Revendications

1. Emballage de cathéter formé comme un tube multicouche pour un cathéter intermittent, dans lequel le tube est un élément en forme de cylindre qui présente une section transversale sensiblement circulaire, et le matériau de tube est flexible et présente une vitesse de transmission de vapeur d'eau équivalente inférieure à 0,50 g/m²/mm/24 heures mesurée en utilisant le procédé WVTR tel qu' il est défini dans la description.

2. Emballage de cathéter selon la revendication 1, dans lequel le matériau de tube présente une vitesse de transmission de vapeur d'eau équivalente inférieure à 0,30 g/m²/mm/24 heures mesurée en utilisant le procédé WVTR tel qu'il est défini dans la description.

3. Emballage de cathéter selon l'une quelconque des revendications précédentes, dans lequel la composition en couches du tube comprend la combinaison d'une ou de plusieurs couche(s) de polymère thermoplastique et d'une ou plusieurs couche(s) d'élastomère thermoplastique.

4. Emballage de cathéter selon l'une quelconque des revendications précédentes, dans lequel la composition en couches du tube comprend deux ou plus de deux couches de polymère thermoplastique.

5. Emballage de cathéter selon l'une quelconque des revendications précédentes, dans lequel la composition en couches du tube comprend deux ou plus de deux couches d'élastomère thermoplastique.

6. Emballage de cathéter selon les revendications 3 et 5, dans lequel la ou les couche (s) d'élastomère thermoplastique comprend/comprennent un copolymère bloc de styrène tel qu'un polystyrène-bloc-poly(éthylène/butylène)-bloc-polystyrène, un polystyrène-bloc-poly(éthylène-propylène)-bloc-polystyrène, un polystyrène-bloc-polybutadiène-bloc-polystyrène ou un polystyrène-bloc-polyisobutylène-bloc-polystyrène.

7. Emballage de cathéter selon la revendication 6, dans lequel la ou les couche(s) d'élastomère thermoplastique comprend/comprennent en outre du polypropylène et/ou du polyéthylène, et optionnellement un agent plastifiant tel que l'huile de paraffine dans un composé.

8. Emballage de cathéter selon l'une quelconque des revendications 3-7, dans lequel la ou les couche(s) d'élastomère thermoplastique comprend/comprennent du polybutène, du polyisobutylène, du caoutchouc butyle durci ou non durci, ou un copolymère bloc avec du polystyrène comme blocs d'extrémité, tels que le styrène-isobutylène-styrène.

9. Emballage de cathéter selon l'une quelconque des revendications 3-7, dans lequel la ou les couche(s) de polymère thermoplastique comprend/comprennent des homopolymères de polyéthylène avec une densité de 0,900 g/cm³ à 0,930 g/cm³.

10. Emballage de cathéter selon l'une quelconque des revendications 3-7, dans lequel la ou les couche(s) de polymère thermoplastique comprend/comprennent des copolymères de polyéthylène avec du butène, de l'hexène ou de l'octène comme comonomères et avec une densité de 0,900 g/cm³ à 0,930 g/cm³.

11. Emballage de cathéter selon l'une quelconque des revendications 3-7, dans lequel la ou les couche(s) de polymère thermoplastique comprend/ un copolymère de polypropylène de 1 % à 25 % d'éthylène comme comonomère distribué soit de façon aléatoire soit sous la forme de blocs discrets et avec une densité de 0,860 g/cm³ à 0,910 g/cm³.

12. Emballage de cathéter selon l'une quelconque des revendications 3-7, dans lequel la ou les couche(s) de polymère thermoplastique comprend/ des copolymères ou des terpolymères de polyéthylène de 1 % à 25 % de vinylacétate, de méthylacrylate, d'éthylacrylate, de butylacrylate, d'acide acrylique, d'acide méthacrylique ou d'acide maléique comme comonomères.

13. Emballage de cathéter selon l'une quelconque des revendications 9 ou 10, dans lequel le polyéthylène est un polyéthylène à faible densité ou un polyéthylène linéaire à faible densité.

14. Emballage de cathéter selon l'une quelconque des revendications précédentes, dans lequel le tube est ondulé.

15. Emballage de cathéter selon l'une quelconque des revendications 1 à 13, dans lequel le tube est droit.

16. Ensemble d'emballage de cathéter, comprenant:
- un emballage de tube,
- un cathéter intermittent,
- un agent de gonflement,
- et optionnellement une poche de collecte d'urine,
dans lequel le tube est un élément en forme de cylindre qui présente une section transversale sensiblement circulaire, et le matériau de tube est flexible et présente une vitesse de transmission de vapeur d'eau inférieure à 0,50 g/m²/mm/24 heures mesurée en utilisant le procédé WVTR tel qu' il est défini dans la description.

17. Ensemble d'emballage de cathéter selon la revendication 16, dans lequel l'agent de gonflement est disposé à l'intérieur de l'emballage de tube.

18. Ensemble d'emballage de cathéter selon l'une quelconque des revendications 16 ou 17, dans lequel l'emballage de tube est du type selon l'une quelconque des revendications 1 à 15.

19. Ensemble d'emballage de cathéter selon l'une quelconque des revendications 16 à 18, dans lequel le cathéter intermittent comprend un revêtement hydrophile.

20. Ensemble d'emballage de cathéter selon l'une quelconque des revendications 16 à 19, dans lequel l'emballage a été stérilisé en utilisant un rayonnement pendant qu'il était en contact avec l'agent de gonflement.

21. Ensemble d'emballage de cathéter selon la revendication 20, dans lequel l'ensemble est stérilisé en utilisant un rayonnement β ou un rayonnement γ.
